# FASCICULE DE BREVET EUROPEEN

(11) **EP 1 450 843 B1**
(45) Date de publication et mention de la délivrance du brevet: **02.08.2006**
(21) Numéro de dépôt: 02800641.9
(22) Date de dépôt: 08.10.2002
(51) Int. Cl.: A61K 38/08, A61K 38/22, A61P 29/00

(54) **UTILISATION DE PEPTIDES ANALOGUES DE LA THYMULINE (PAT) POUR LA FABRICATION DE MEDICAMENTS CONTRE LA DOULEUR**
VERWENDUNG VON THYMULINÄHNLICHEN PEPTIDEN ZUR HERSTELLUNG VON SCHMERZMITTELN
USE OF THYMULIN-LIKE PEPTIDES FOR MAKING PAIN-RELIEVING MEDICINES

(30) Priorité: 09.10.2001 FR 0112984
(43) Date de publication de la demande: 01.09.2004
(73) Titulaire: INSTITUT NATIONAL DE LA SANTE ET DE LA RECHERCHE MEDICALE (INSERM), 75654 Paris Cédex 13 (FR)
(72) Inventeur: DARDENNE, Mireille, F-75015 Paris (FR); PLEAU, Jean-Marie, F-91120 Palaiseau (FR); BACH, Jean-François, F-75007 Paris (FR); SAADE, Nayef, Beyrouth (LB); SAFIEH-GARABEDIAN, Bared, c/o Geminex, 1st floor, P.O. Box 16-5734 Beirut (LB)
(74) Mandataire: Peaucelle, Chantal
(86) Numéro de dépôt international: PCT/FR2002/003428
(87) Numéro de publication internationale: WO 2003/030927

(56) Documents cités:
- EP-A- 0 041 019
- US-A- 5 112 810
- US-A- 5 288 706
- DATABASE WPI Section Ch, Week 199610 Derwent Publications Ltd., London, GB; Class B04, AN 1996-094122 XP002211536 & JP 08 003057 A (MITSUI TOATSU CHEM INC), 9 janvier 1996 (1996-01-09)
- DATABASE WPI Section Ch, Week 199432 Derwent Publications Ltd., London, GB; Class B04, AN 1994-260440 XP002211537 & JP 06 192120 A (MITSUI TOATSU CHEM INC), 12 juillet 1994 (1994-07-12)
- SAFIEH-GARABEDIAN BARED ET AL: "Potent analgesic and anti- inflammatory actions of a novel thymulin -related peptide in the rat." BRITISH JOURNAL OF PHARMACOLOGY, (2002 JUL) 136 (6) 947-55. , XP008015703
- SAFIEH-GARABEDIAN B ET AL: "Cytokine-mediated or direct effects of thymulin on the nervous system as assessed by pain -related behavior." NEUROIMMUNOMODULATION, (1999 JAN-APR) 6 (1-2) 39-44. REF: 34, XP008005719 cité dans la demande

## Description

L'invention se rapporte à l'utilisation de peptides analogues de la thymuline pour la fabrication de médicaments contre la douleur.

La plupart des douleurs aiguës ou chroniques résultent d'une réaction inflammatoire. Les traitements préconisés pour diminuer la douleur consistent souvent à réduire en premier lieu la réaction inflammatoire.

Il existe actuellement deux classes principales de médicaments anti-inflammatoires :
- les anti-inflammatoires non stéroïdiens (NSAID),
- les corticostéroïdes.

Les NSAID et les corticostéroïdes présentent l'inconvénient d'associer à leur effet thérapeutique bénéfique (diminution de l'inflammation et de la douleur) un effet secondaire gênant.

En effet, les NSAID provoquent la formation d'ulcères tandis que les corticostéroïdes ont une action immunosuppressive.

Le médicament analgésique anti-inflammatoire idéal serait donc un médicament ne présentant pas d'effet secondaire, ni sur les fonctions physiologiques, ni sur le système immunitaire.

Par ailleurs, il existe un second type de douleur n'ayant pas pour origine l'inflammation. Ces douleurs neurogènes sont, entre autres, caractérisées par leur résistance aux traitements traditionnels, y compris aux opiacées. Différents traitements ont été envisagés tels que l'utilisation d'anti-inflammatoires, d'anti-épileptiques, d'antidépresseurs, de drogues sympatholytiques, ou des combinaisons de ceux-ci.

Cependant, les douleurs neurogènes sont très variées et donc très difficilement traitables.

Il résulte de ces considérations que les médicaments aujourd'hui disponibles pour soigner la douleur sont en nombre limité et se révèlent parfois inefficaces. Cette inefficacité peut en outre résulter d'une accoutumance au produit. Le praticien se trouve alors obligé de modifier sa prescription. Pour que celle-ci se révèle efficace, il doit pouvoir disposer d'une autre classe de médicaments.

Ceci explique l'importance de la recherche dans ce domaine.

Des peptides analogues de la thymuline de la présente invention ont déjà été décrits dans les brevets et certificat d'addition FR 7715963, FR 7811870 et EP 0041019 en tant que médicaments pour le traitement de maladies auto-immunes, la stimulation de cellules T et la prévention de rejet de greffes. Les propriétés de ces peptides sur le système immunitaire se sont révélées zinc-dépendantes. En effet, le zinc contenu dans le peptide lui donne une conformation tétraédrique qui correspond à la forme active de la molécule. En l'absence de zinc, les peptides analogues ne possédaient plus aucune activité. De plus, il s'est avéré par la suite que ces propriétés, mises en évidence par essais *in vitro,* n'ont eu aucun effet sur le système immunitaire lors des essais *in vivo*. Ils n'ont pas, par ailleurs, provoqué d'effets secondaires. Ces peptides présentent une parfaite innocuité.

De nombreuses publications ont montré que la thymuline pouvait, selon la dose injectée, provoquer ou réduire une hyperalgésie (Safieh-Garabedian et al., *Neuroimmunomodulation*, **6** :39-44, 1999). A faible dose (de l'ordre du nanogramme par rat, soit 0,2 à 20µg/Kg), la thymuline induit l'hyperalgésie, tandis qu'à des doses plus importantes (de l'ordre du microgramme par rat, soit 50-100µg/Kg), elle la réduit. L'utilisation de la thymuline était donc impossible étant donné son effet sur le système immunitaire et cet effet dose-dépendant (ou biphasique), provoquant la douleur ou la diminuant.

Les inventeurs se sont alors intéressés aux peptides analogues de la thymuline inactifs vis-à-vis du système immunitaire. Bien que ceux-ci n'aient pas présenté l'activité première escomptée, ils ont vérifié leur spectre d'activité et constaté que, contre toute attente, ils ne présentaient pas cet effet ambivalent dose dépendant, qu'ils possédaient uniquement une activité analgésiante, sans être zinc dépendant, et qu'enfin, ils se révélaient actifs *in vivo*.

Les travaux des inventeurs les ont alors conduits à élaborer, à partir de ces peptides dont l'innocuité a déjà par ailleurs été établie, une nouvelle classe de médicaments anti-douleur permettant de traiter les douleurs d'origine inflammatoire et/ou les douleurs neurogènes.

La présente invention se rapporte à l'utilisation de peptides analogues de la thymuline (PAT) inactifs vis-à-vis du système immunitaire ne comportant pas de zinc et présentant une activité anti-douleur pour fabriquer un médicament destiné au traitement de la douleur.

Par « peptides analogues de la thymuline inactifs vis-à-vis du système immunitaire », on entend des peptides analogues de la thymuline inactifs vis-à-vis des réponses immunitaires spécifiques des lymphocytes T. En particulier, ces peptides ne forment pas de complexes avec des métaux tel que le zinc, par exemple. (Dardenne et al., *PNAS,* **79** : 5370-5373, 1982)

L'utilisation des peptides selon l'invention présente l'avantage d'être efficace contre la douleur sans induire d'effets secondaires gênants.

Ces peptides sont par ailleurs efficaces à des doses 10 à 100 fois inférieures à celles des analgésiques classiques, par exemple, chez le rat, les doses utilisées sont de l'ordre de 1µg par rat (soit 5µg/Kg) contre 4mg/Kg pour les drogues anti-inflammatoires non stéroïdiennes et 200mg/Kg pour les drogues anti-inflammatoires stéroïdiennes. Une activité a également été constatée à des doses inférieures, de l'ordre de 50 à 200 ng/rat, soit 0,25 à 1µg/Kg.

En particulier, les peptides utilisés selon l'invention, présentent la séquence suivante :
X-Gln-Gly-Gly-Ser-Asn
dans laquelle X représente Ser, Lys-Ser, Ala-Lys-Ser, Glu-Ala-Lys-Ser, Gln-Ala-Lys-Ser, PyroGlu-Ala-Lys-Ser, ainsi que toute séquence dérivée comportant 1 ou 2 acides aminés modifiés, les modifications possibles étant de la nature suivante :
- PyroGlu :: D-PyroGlu, Glu, Gln
- Gln :: Z-Gln, D-Gln , Pro, Cys (S-CONH₂) , Met (O), Glu, Glu (γ-cyano), Glu (γCS-NH₂) , D-Glu, Asn, NorVal
- Ala :: D-Ala, Z-Ala, Ac-Ala
- Lys :: Arg, D-Lys, N-γ-Z-Lys, Lys(N⁶ acetyl), Orn, Har, 2-amino-hexanoyl, 2,6-diamino-hexynoyl, 2,6-diamino-hexenoyl, Hep, D-Lys(N⁶-acétyl)
- Ser :: Ala, (N-méthyl)Ser, D-Ser, Thr
- Gly :: Ala, Ser, D-Ala, D-Leu
- Asn :: CyanoAla, Thio-Asn, Asp, Gln, Glu, β-Ala-NH₂, D-Asn, Asn-NH₂

La référence aux « analogues de la thymuline » exclut clairement la thymuline, peptide présentant la séquence suivante : PyroGlu-Ala-Lys-Ser-Gln-Gly-Gly-Ser-Asn.

L'invention vise en particulier à protéger l'utilisation des peptides suivants :
(1) PyroGlu-Ala-Lys-Ser-Gln-Gly-Gly-Ser-Asp
(2) PyroGlu-Ala-Lys-Ala-Gln-Gly-Gly-Ser-Asn
(3) PyroGlu-Ala-Lys-Ser-Gln-Gly-Gly-Ser-Gln
(4) PyroGlu-Ala-Lys-Ser-Gln-Gly-Gly-Ser-β-Ala-NH₂
(5) PyroGlu-Ala-Lys-Ser-Gln-Gly-Gly-Ser-D-Asn
(6) PyroGlu-Ala-Lys-Ser-Gln-Gly-Gly-Ser-Asn-NH₂
(7) PyroGlu-Ala-Lys-Ser-Asn-Gly-Gly-Ser-Asn
(8) PyroGlu-Ala-Lys-Ser-Nva-Gly-Gly-Ser-Asn
(9) PyroGlu-Ala-Lys-Ser-Gln-Gly-Gly-Ala-Asp
(10) Gln-Ala-Lys-Ser-Gln-Gly-Gly-Ser-Asp
(11) Gln-Ala-Lys-Ala-Gln-Gly-Gly-Ser-Asn
(12) Gln-Ala-Lys-Ser-Gln-Gly-Gly-Ser-Gln
(13) Gln-Ala-Lys-Ser-Gln-Gly-Gly-Ser-β-Ala-NH₂
(14) Gln-Ala-Lys-Ser-Gln-Gly-Gly-Ser-D-Asn
(15) Gln-Ala-Lys-Ser-Gln-Gly-Gly-Ser-Asn-NH₂
(16) Gln-Ala-Lys-Ser-Asn-Gly-Gly-Ser-Asn
(17) Gln-Ala-Lys-Ser-Nva-Gly-Gly-Ser-Asn
(18) Gln-Ala-Lys-Ser-Gln-Gly-Gly-Ala-Asp
(19) Glu-Ala-Lys-Ser-Gln-Gly-Gly-Ser-Asp
(20) Glu-Ala-Lys-Ala-Gln-Gly-Gly-Ser-Asn
(21) Glu-Ala-Lys-Ser-Gln-Gly-Gly-Ser-Gln
(22) Glu-Ala-Lys-Ser-Gln-Gly-Gly-Ser-β-Ala-NH₂
(23) Glu-Ala-Lys-Ser-Gln-Gly-Gly-Ser-D-Asn
(24) Glu-Ala-Lys-Ser-Gln-Gly-Gly-Ser-Asn-NH₂
(25) Glu-Ala-Lys-Ser-Asn-Gly-Gly-Ser-Asn
(26) Glu-Ala-Lys-Ser-Nva-Gly-Gly-Ser-Asn
(27) Glu-Ala-Lys-Ser-Gln-Gly-Gly-Ala-Asp
(28) Ala-Lys-Ser-Gln-Gly-Gly-Ser-Asp
(29) Ala-Lys-Ala-Gln-Gly-Gly-Ser-Asn
(30) Ala-Lys-Ser-Gln-Gly-Gly-Ser-Gln
(31) Ala-Lys-Ser-Gln-Gly-Gly-Ser-β-Ala-NH₂
(32) Ala-Lys-Ser-Gln-Gly-Gly-Ser-D-Asn
(33) Ala-Lys-Ser-Gln-Gly-Gly-Ser-Asn-NH₂
(34) Ala-Lys-Ser-Asn-Gly-Gly-Ser-Asn
(35) Ala-Lys-Ser-Nva-Gly-Gly-Ser-Asn
(36) Ala-Lys-Ser-Gln-Gly-Gly-Ala-Asp
(37) Lys-Ser-Gln-Gly-Gly-Ser-Asp
(38) Lys-Ala-Gln-Gly-Gly-Ser-Asn
(39) Lys-Ser-Gln-Gly-Gly-Ser-Gln
(40) Lys-Ser-Gln-Gly-Gly-Ser-β-Ala-NH₂
(41) Lys-Ser-Gln-Gly-Gly-Ser-D-Asn
(42) Lys-Ser-Gln-Gly-Gly-Ser-Asn-NH₂
(43) Lys-Ser-Asn-Gly-Gly-Ser-Asn
(44) Lys-Ser-Nva-Gly-Gly-Ser-Asn
(45) Lys-Ser-Gln-Gly-Gly-Ala-Asp

Plus spécifiquement, l'invention vise à protéger l'utilisation des peptides suivants :
(19) Glu-Ala-Lys-Ser-Gln-Gly-Gly-Ser-Asp
(1) PyroGlu-Ala-Lys-Ser-Gln-Gly-Gly-Ser-Asp
(10) Gln-Ala-Lys-Ser-Gln-Gly-Gly-Ser-Asp
(28) Ala-Lys-Ser-Gln-Gly-Gly-Ser-Asp
(37) Lys-Ser-Gln-Gly-Gly-Ser-Asp

Ces peptides ont été étudiés dans deux modèles d'inflammation et d'hyperalgésie chez le rat, induits soit par des injections intraplantaires (localisées), soit par des injections intrapéritonéales (systémiques) d'endotoxines. Le prétraitement par ces peptides abolit, de manière dose-dépendante, à la fois l'hyperalgésie mécanique et l'hyperalgésie thermique. Par ailleurs, le prétraitement réduit de façon significative l'hyperproduction d'IL-1β, d'IL-6, de TNFα et de NGF due à une injection intraplantaire d'endotoxine. Dans le cas d'une injection intrapéritonéale, ayant pour conséquence un état comparable au choc septique (douleur, fièvre, somnolence et anorexie), le prétraitement prévient l'hyperalgésie et maintient le corps à une température normale.

Enfin, ces peptides ont des effets analgésiques identiques ou supérieurs à ceux des autres drogues anti-inflammatoires, tout en n'induisant aucun changement évident des paramètres physiologiques ou comportementaux, pour toutes les doses utilisées.

Ces peptides présentent donc des propriétés analgésiques et/ou anti-inflammatoires.

Les propriétés analgésiques de ces peptides s'étendent également aux douleurs neurogènes (ou douleurs neuropathiques). Plusieurs modèles ont été utilisés pour tester des douleurs neurogènes d'origines différentes : deux modèles animaux de mononeuropathie, un autre modèle de douleur de type somatique ou viscéral. Ces modèles ont permis de constater une réduction significative de l'allodynie mécanique, et parfois, de l'allodynie thermique. Les manifestations neuropathiques se trouvent inhibées, tandis que le comportement lié à la douleur induit par l'injection de substances irritantes (capsaïcine) est réduit. Dans tous les cas, ces peptides présentent des effets inhibiteurs aussi importants ou plus importants que ceux induits par les autres traitements utilisés dans le cas des douleurs neurogènes.

Compte tenu de leurs propriétés, l'utilisation de ces peptides est plus particulièrement préconisée dans le traitement de migraines, sciatiques, neuropathies et les douleurs d'origine inflammatoires aigües ou chroniques.

Pour une efficacité optimum, les doses d'administration doivent être comprises entre 0,01 et 1 mg/kg, l'administration s'effectuant par les voies les plus variées y compris parentérale, transcutanée ou nasale.

D'autres aspects et avantages de l'invention seront mieux compris au vue des exemples suivants et en se référant aux figures qui représentent, respectivement:
- la figure 1 illustre la diminution, dose-dépendante de l'hyperalgésie induite par une injection d'endotoxines,
- la figure 2 présente une étude comparative des effets anti-hyperalgésiques des peptides selon l'invention avec des stéroïdes, des anti-inflammatoires non stéroidiens et des tripeptides analgésiques,
- la figure 3 illustre les effets anti-inflammatoires d'un prétraitement avec les peptides selon l'invention, par réduction des concentrations de cytokines pro-inflammatoires et de NGF,
- la figure 4 présente les effets du prétraitement sur la douleur (A et B) et sur la fièvre (C) provoqués par l'injection systémique d'endotoxines simulant le choc septique,
- la figure 5 illustre l'inhibition de l'allodynie (douleur anormale provoquée par des stimuli inoffensifs) et de l'hyperpathie (réaction exagérée à une stimulation nociceptive d'intensité modérée) chez des rats présentant une mononeuropathie CCI (par constriction chronique du nerf sciatique), prétraités avec les peptides selon l'invention,
- la figure 6 illustre l'inhibition de l'allodynie et de l'hyperpathie chez des rats présentant une mononeuropathie SNI (douleur du nerf épargné d'une patte dénervée), prétraités avec les peptides selon l'invention,
- la figure 7 présente une étude de posologie des peptides selon l'invention, sur des rats présentant les deux types précités de mononeuropathie,
- la figure 8 présente une étude de posologie des peptides selon l'invention, sur des rats présentant une hyperalgésie induite par injection de capsaïcine (douleur neurogène somatique) ,
- la figure 9 illustre l'atténuation, par les peptides selon l'invention, de la douleur viscérale induite par une injection intrapéritonéale de capsaïcine (douleur neurogène spasmodique viscérale) ,
- la figure 10 illustre la comparaison de différentes drogues sur la neuropathie, et,
- la figure 11 présente les résultats d'un traitement quotidien par PAT.

### Exemple 1 : Propriétés analgésiques et anti-inflammatoires des peptides selon l'invention.

### 1. Matériels et méthodes .

Les expériences ont été menées sur des rats Sprague-Dawley adultes, mâles, de 200 à 250 g. Les animaux ont été élevés dans des conditions optimums de lumière et de température (cycle de 12h de lumière et de pénombre, 22 ± 3°C). La nourriture et l'eau étaient dispensées ad libitum. Toutes les expériences ont été menées dans le respect des directives éthiques relatives aux expérimentations sur la douleur menées sur animaux à l'état conscient (Zimmermann M., **1983,** *Ethical guidelines for investigations of experimental pain in conscious animals,* Pain, **16**:109-110) et elles ont été approuvées par le comité de l'institution pour les soins portés aux animaux.

### Mesures comportementales

Des tests de douleurs thermiques et mécaniques ont été effectués durant les 3 jours consécutifs avant les injections, afin d'établir un état de référence.

Le seuil de nociception mécanique a été déterminé par le test de pression mécanique sur la patte (PP), tandis que celui de nociception thermique a été déterminé par les tests de la plaque chauffante (HP), d'immersion de la patte dans l'eau chaude (PI) et de flexion de la queue (TF).

Le test PP est effectué en appliquant une pression constante de 0,20 g/cm² alternativement sur les pattes arrières gauche et droite avec un intervalle de 5 minutes entre 2 pressions consécutives. La pression est arrêtée quand l'animal présente une réaction typique caractérisée par un vigoureux réflexe de flexion.

Dans le test HP, les animaux sont placés individuellement sur une plaque chauffante (52,5°C ± 0,3°C). Le seuil de douleur est mesuré par le temps de latence écoulé entre le moment òu l'animal est placé sur la plaque chauffante et le premier signe de douleur, indiqué par le fait que l'animal se lèche la patte ou saute.

Dans le test PI, les pattes arrières sont trempées alternativement dans de l'eau distillée à 48°C et le temps de latence écoulé jusqu'au premier signe de retrait de la patte est enregistré.

Dans le test TF, la queue de chaque animal est immergée dans de l'eau distillée à 50,5°C. Le temps de latence mis par l'animal pour l'en retirer est enregistré. Les résultats sont basés sur 3 essais consécutifs effectués à 5 minutes d'intervalle.

### Administration des drogues

L'hyperalgésie inflammatoire a été établie à partir de deux modèles animaux : l'un pour une inflammation localisée, l'autre pour une inflammation systémique.

Dans le modèle dit localisé, les rats ont reçu une injection intraplantaire d'une solution (1,25 *µ*g dans 50 *µ*l de sérum physiologique à 9%) d'endotoxines (lipopolysaccharides de la *Salmonella typhasa*, *Sigma)* dans une des pattes arrières, ce qui provoque à la fois une hyperalgésie thermique et mécanique, restreinte à la patte ayant subi l'injection.

Dans le second modèle, les rats subissent une injection intrapéritonéale d'endotoxines (25 *µ*g dans 100 *µ*l de sérum physiologique).

Divers traitements sont ensuite possibles :
Protocole 1(a) : différents groupes de rats (n = 5 dans chaque groupe) ont été traités avec le peptide analogue (PAT) Glu-Ala-lys-Ser-Gln-Gly-Gly-Ser-Asp (synthétisé par Quantum Biotechnologies Inc., Canada), de la manière suivante :
   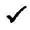 soit, ils ont reçu une injection intrapéritonéale (25 *µ*g dans 50 *µ*l de sérum physiologique) de ce peptide,
   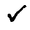 soit, ils ont été prétraités avec différentes doses de ce peptide (1,5 et 25 *µ*g dans 50 *µ*l de chlorure de sodium) par injection intrapéritonéale, 30 minutes avant l'injection d'endotoxines (ET) (1,25 *µ*g dans 50 *µ*l de sérum physiologique, en injection intraplantaire).
Protocole 1(b) : d'autres expériences ont eu pour but de comparer l'efficacité des peptides analogues avec celle des stéroïdes, NSAID et des peptides connus pour leur antagonisme de l'hyperalgésie induite par IL-1β et les prostaglandines.

Un groupe de rats a été prétraité avec des injections intrapéritonéales de Lys-D-Pro-Thr (10 mg/kg) dans 100*µ*l de sérum physiologique, 30 minutes avant l'injection de PAT. Ce tripeptide est connu son antagonisme de l'hyperalgésie induite par IL-1β seulement.

Dans le second groupe, les rats ont reçu des injections intrapéritonéales du tripeptide Lys-D-Pro-Val, (10 mg/kg) dans 100 *µ*l de solution saline, 30 minutes avant l'injection de PAT. Lys-D-Pro-Val est un antagoniste de l'hyperalgésie induite par IL-1β et PGE₂.

Les doses utilisées pour ces tripeptides sont celles décrites dans l'article de Safieh-Garabedian (Safieh-Garabedian B., Kanaan S.A., Haddad J.J., Abou Jaoude P., Jabbur S.J., Saade N.E. **1997,** *Involvement of interleukin-1β, nerve growth factor and prostaglandin-E2 in endotoxin induced localized inflammatory hyperalgesia.* Brit. J. Pharmacol. 121 : 1619-1626).

Un troisième et un quatrième groupe ont été traités par la dexaméthasone et de l'indométhacine.

La dexaméthasone phosphate dissoute dans une solution de sodium a 9 %o été injectée, à la concentration de 200 *µ*g/kg, juste avant et 3 heures après l'injection de ET.

L'indométhacine a été préparée en dissolvant de l'indométhacine lactose dans une solution saline tampon (pH 7,4) et a été injectée, à la concentration de 4 mg/kg, juste avant et 3 heures après l'injection d'ET.

Tous les tests des expériences ci-dessous ont été effectués 9 heures après l'injection d'ET. Ceci coïncide avec le pic d'hyperalgésie dans ce modèle. Les injections de solution saline (50-100 *µ*l en intraplantaire) n'ont pas montré d'altération significative du seuil de la douleur.

Protocole 2 (a) : Un groupe de rats a reçu une injection intrapéritonéale d'ET (50 *µ*g) tandis que l'autre groupe a été prétraité avec le peptide analogue à la thymuline (PAT) (25 *µ*g, injection intrapéritonéale), 30 minutes avant l'injection d'ET.

Les tests TF et PP ont alors été effectués 1 heure, 3 heures et 6 heures après l'injection d'ET.

Protocole 2(b) : Différents groupes de rats (n = 5 dans chaque groupe) ont été traités de la manière suivante :
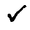 soit, ils ont reçu une injection intrapéritonéale de 50 *µ*g d'ET,
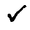 soit, ils ont été prétraités avec le peptide PAT (25 *µ*g, injection intrapéritonéale), 30 minutes avant une injection d'endotoxine.

La température rectale a été mesurée à 1 heure, 3 heures et 6 heures.

Un groupe contrôle a reçu une injection intrapéritonéale de peptide PAT (25 *µ*g dissous dans 100*µ*l de solution saline) .

### Cytokines et facteur de croissance nerveuse (NGF)

Ces expériences ont nécessité le prélèvement de tissus. Les animaux sont anesthésiés jusqu'à la mort (penthiobarbital de sodium, 50 mg/kg) et la peau des pattes arrières est prélevée soit 1h (pour la détermination du niveau de TNFα) soit 4h (pour la détermination d'IL 1β, IL 6 et NGF) après l'injection d'endotoxine.

Les échantillons de tissus sont pesés puis rapidement congelés et stockés à -70°C en vue de procéder à l'évaluation de IL-1β, TNF-α, IL-6 et NGF.

Dans une autre série d'expériences, les tissus sont prélevés comme décrit ci-dessus, sur différents groupes de rats, les uns prétraités avec le PAT, 30 minutes avant l'injection d'ET, les autres ayant seulement été injectés avec PAT.

Les tissus sont homogénéisés dans une solution tampon phosphate (PBS, pH = 7,4), contenant 0,4 M de NaCl , 0,05% de Tween-20®, 0,5% d'albumine de sérum bovin (BSA), 0,1 mM de fluorure de phénylméthylsulfonyle, 0,1 mM de chlorure de benzéthonium, 10 mM d'EDTA et 20 KI/mL d'aprotinine.

Le mélange est ensuite centrifugé à 1 200 g pendant 60 minutes à 4°C. Les cytokines et le NGF contenus dans le surnageant ont été mesurés par les tests ELISA.

Le NGF est mesuré à l'aide d'un kit immunologique (Promega) en suivant les instructions recommendées par le fabriquant.

La mesure des cytokines IL-1β, TNF-α et IL-6 a été effectuée selon les protocoles décrits par Safieh-Garabedian (Safieh-Garabedian B., Dardenne M., Kanaan S.A., Atweh S.F., Jabbur S.J., Saadé N.E. **2000**. *The role of cytokines and prostaglandin-E2 in thymulin induced hyperalgesia.* Neuropharmacology 39 :1653-1661).

### Analyse statistique et traitement des données

Une valeur de seuil de douleur, pour les différents tests de la nociception, a été définie pour chaque groupe d'animaux. Les données obtenues pour chaque drogue testée ont été comparées soit avec le témoin établi avant l'injection soit avec deux types de témoins : une série d'animaux ayant subi une injection d'ET et une autre série d'animaux ayant subi une injection de solution de chlorure de sodium à 9‰.

Pour l'évaluation des niveaux de cytokines et de NGF, les valeurs obtenues sur des animaux injectés avec ET seulement, drogue seulement ou ET plus drogue ont été comparées aux valeurs obtenues sur des groupes d'animaux contrôles injectés avec une solution de chlorure de sodium à 9%.

L'importance de l'écart type a été établie par ANOVA suivie d'un test de Bonferroni.

### 2. Résultats

### Effet du peptide PAT sur l'hyperalgésie inflammatoire provoquée par l'injection intraplantaire d'ET

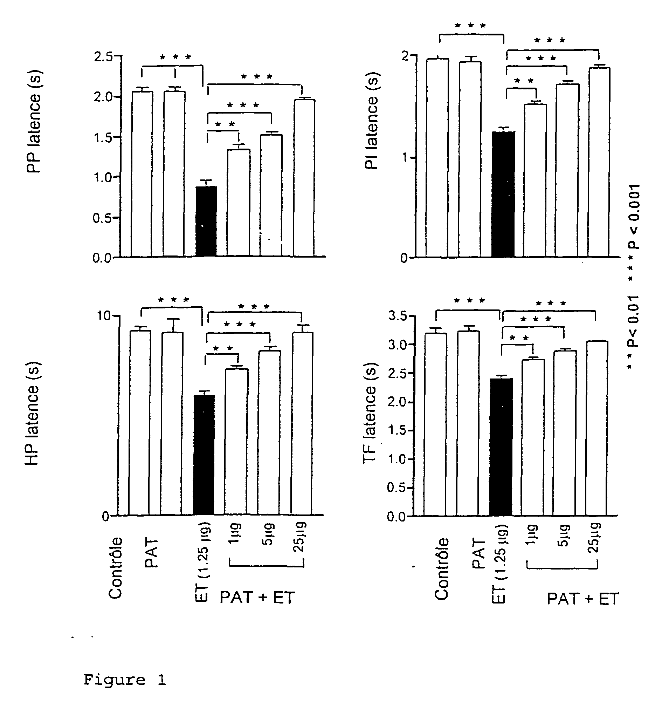

L'injection intraplantaire d'ET (1,25 *µ*g dans 50 *µ*l de solution saline) dans la patte arrière de rats a entraîné une diminution significative des seuils nociceptifs mesurés à 9 h (pic d'hyperalgésie) d'après le test PP (0,87±0,09 s contre 2,06±0,06 s pour la solution saline de contrôle, P<0,001) pour l'hyperalgésie mécanique et d'après les tests PI (1,25±0,04 s contre 1,97±0,05 s pour la solution saline de contrôle, P<0,001), HP (6,0±0,18 contre 9,24±0,16 s pour la solution saline de contrôle, P<0,001) et TF (2,40±0,06 s contre 3,19±0,08 s pour la solution saline de contrôle, P<0,001) pour l'hyperalgésie thermique. Le traitement par le peptide PAT(1,5 et 25 *µ*g) a diminué de manière dose dépendante l'hyperalgésie provoquée par l'injection d'ET (fig. 1). Avec la dose de 25 *µ*g de PAT, les latences de déclenchement des diverses réponses ont été de 2,05 ± 0,07 s, 1,94 ± 0,005 s, 9,12 ± 0,7 s et 3,22 ± 0,09 s pour les tests PP, PI, HP et TF respectivement (p>0,05 pour toutes les valeurs en comparaison avec l'état basal ou avec les valeurs obtenues avec la solution saline). L'injection intrapéritonéale de PAT seule (25 *µ*g dans 50 *µ*l de solution saline) n'a pas entraîné de modification significative des temps de latence aux différents tests de douleur.

### Comparaison de l'efficacité du peptide PAT avec d'autres médicaments et analogues

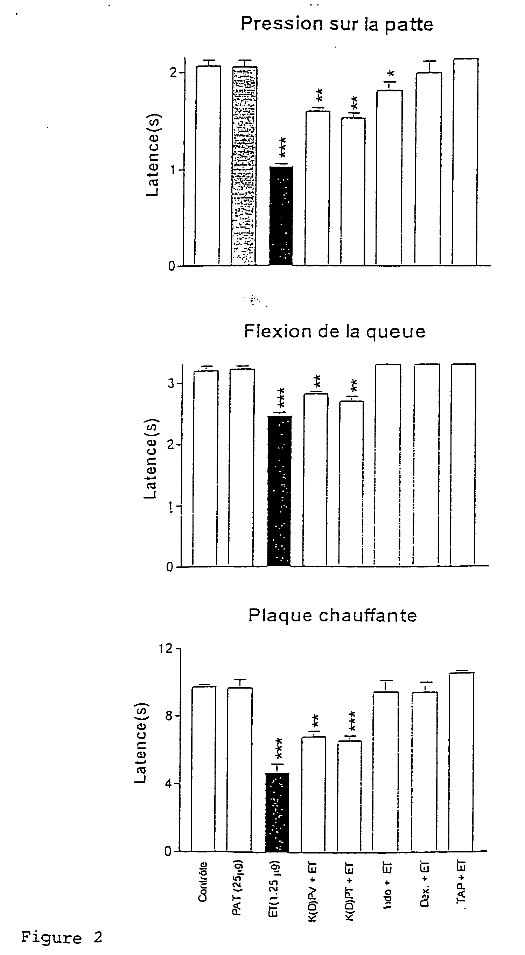

Lorsque l'on compare l'effet de PAT sur l'hyperalgésie provoquée par une injection intraplantaire d'ET (1,25 *µ*g) aux effets obtenus avec un stéroïdien, un NSAID et les peptides Lys-D-Pro-Val et Lys-D-Pro-Thr, les résultats obtenus démontrent que PAT est un agent analgésique beaucoup plus efficace que les peptides Lys-D-Pro-Val et Lys-D-Pro-Thr (fig. 2). PAT a des effets similaires à ceux de l'indométhacine et de la dexaméthasone, mais à des concentrations bien moindres (fig. 2).

### Effet de PAT sur les cytokines

L'injection d'endotoxines dans la patte arrière de rats a entraîné une augmentation significative (P<0,001) des concentrations de cytokines pro-inflammatoires et de NGF en comparaison avec des rats auxquels avait été injecté de la solution saline, ou avec les pattes des mêmes rats n'ayant reçu aucun produit. Une heure après injection d'ET, la concentration de TNF-α était de 345,0 ± 61,0 pg/patte contre 100,0 ± 8,00 pg/ patte après injection de solution saline de contrôle. Trois heures après injection d'ET, la concentration d'IL-1β était de 2850,6±255,4 pg/patte contre 400,0 ± 45,0 pg/patte après injection de la solution saline de contrôle, la concentration d'IL-6 était de 2831,0 ± 285,0 pg/patte contre 250,0 ± 50,0 pg/patte pour la solution saline de contrôle et la concen-tration de NGF était de 23,0 ± 1,73 ng/patte contre 9,11 ± 1,6 ng/patte pour la solution saline de contrôle.

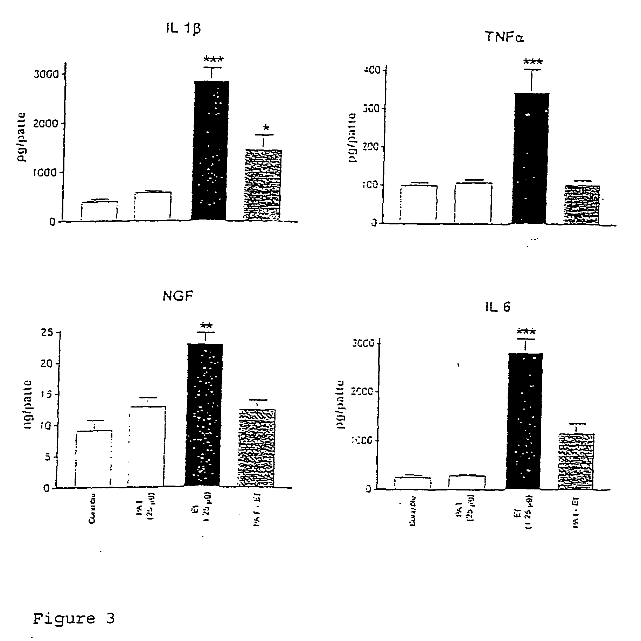

Le traitement préalable par PAT a annulé l'augmentation de la concentration de TNF-α et a réduit de manière significative les concentrations d'IL-1β (de 2850,6 ± 255,4 à 1686,0 ± 266,0 pg/patte, P < 0,01), d'IL-6 (de 2831 ± 285 à 1158 ± 197,0 pg/patte, P < 0,001) et de NGF (de 23,0 ± 1,73 à 16,73 ± 2,70 ng/patte, P < 0,001) (fig. 3A, B, C et D). Des injections de PAT (25 *µ*g) sur des animaux de contrôle n'ont pas entraîné de modification significative des concentrations de cytokines ni de NGF, comme le montre la fig. 3.

### Effet de PAT sur l'hyperalgésie inflammatoire systémique provoquée par l'ET.

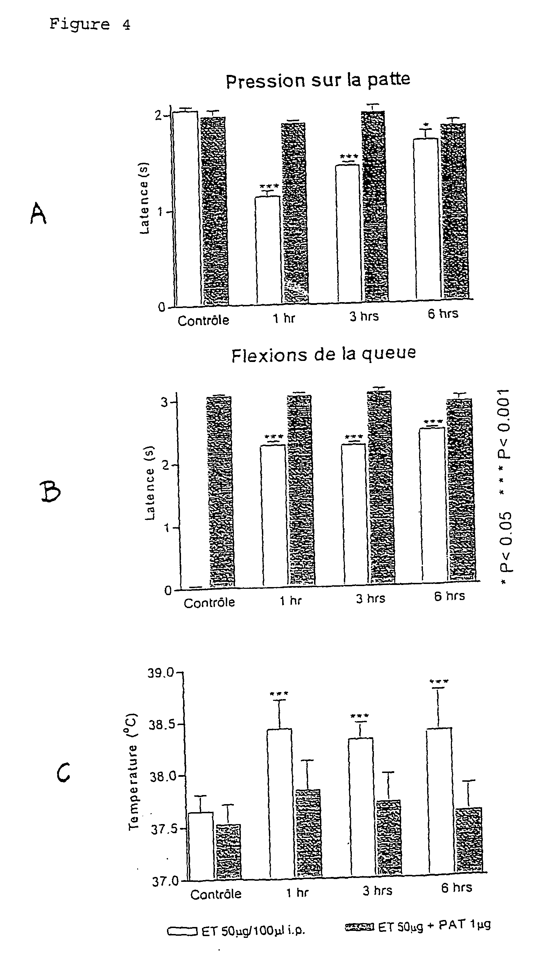

Des injections d'ET (50 *µ*g, i.p.) ont entraîné une diminution significative des seuils nociceptifs à une heure d'après le test PP (1,13 ± 0,06 contre 2,03 ± 0,04 s pour les contrôles, P < 0,001) et d'après le test TF (2,27 ± 0,06 contre 3,08 ± 0,04 s pour les contrôles, P<0,001). L'hyperalgésie est restée évidente pendant six à neuf heures après l'injection (fig. 4A et 4B). Un traitement préalable par injection intrapéritonéale de PAT (25 *µ*g) trente minutes avant administration systémique d'ET a aboli l'hyperalgésie mécanique (fig. 4A) et l'hyperalgésie thermique (fig. 4B) produites par l'ET.

L'endotoxine est un pyrogène connu et son injection a entraîné une augmentation significative de la température corporelle au bout d'une heure (38,43±0,028°C contre 37,65±0,16°C pour les contrôles). La température est ensuite restée significativement élevée à six heures (fig. 4C). Un traitement préalable par PAT en injection intrapéritonéale (25 *µ*g) a annulé le processus d'élévation de la température corporelle provoqué par ET et les valeurs n'ont pas été significativement différentes de celles des contrôles (fig. 4C) .

### Exemple 2 : Propriétés analgésiques d'un peptide selon l'invention (PAT) sur la douleur d'origine neurogène.

### 1. Matériels et méthodes

Des rats Sprague-Dawley adultes mâles (250-300 g) ont été utilisés pour ces essais. Pendant la période d'expérimentation, les rats ont été placés dans des conditions standard (4 à 5 individus par cage, cycle jour/nuit de 12 heures, 22,2°C), avec accès libre à de l'eau et à de la nourriture. Les procédures chirurgicales nécessaires ont été pratiquées sous anesthésie profonde par la kétamine (Ketalar® , 40-50 mg/kg, injection intrapéritonéale), précédée d'une pré-anesthésie à la chlorpromazine (8 mg/kg, idem) et à l'atropine (0,05 mg/kg, idem).

Cette étude a été basée sur deux protocoles expérimentaux pour l'induction de la douleur d'origine neurogène. Le premier protocole a utilisé deux modèles animaux de mononeuropathie. Le second protocole a été basé sur l'injection de capsaïcine, substance connue pour activer des groupes spécifiques de fibres afférentes impliquées dans la signalisation nociceptive.

### a) Protocole I : modèles animaux de mononeuropathie

### Induction de la mononeuropathie :

La mononeuropathie a été provoquée dans des groupes de rats différents (n=6 rats dans chaque groupe) selon le modèle CCI (chronic constriction injury, constriction chronique du nerf, Bennet G.J. et Xie Y.K., **1988,** *A peripheral mononeuropathy in rat that produces disorders of pain sensation like those seen in man, Pain,* **33**:87-107) ou selon le modèle SNI (spared nerve injury, nerf épargné de la patte dénervée, Decosterd I. et Woolf C.J., **2000**, *Spared nerve injury : an animal model of persistent peripheral neuropathic pain, Pain,* **87:**149-158). Le nerf sciatique a été exposé après section de la partie postérieure de la cuisse et par incision à travers la peau et la couche de graisse recouvrant le creux poplité. Pour le modèle CCI, quatre ligatures lâches (catgut chromé 4,0) ont été placées du côté proximal de la trifurcation sciatique. Pour le modèle SNI, le nerf sciatique poplité externe et le nerf sciatique poplité interne ont été isolés, étroitement ligaturés et sectionnés, tandis que le nerf sural innervant l'aspect latéral de la patte est resté intact.

### Tests comportementaux :

Les rats ont été placés dans des compartiments individuels d'une cage dont le sol était fait en grillage métallique permettant d'atteindre le coussinet plantaire et l'aspect latéral de la patte avec des filaments.

Pour l'allodynie mécanique, la surface plantaire des pattes arrières (surface latérale pour le modèle SNI et surface plantaire médiane pour le modèle CCI) a été touchée avec des filaments de Von Frey (VFF 4,31 et 5,07, Stoelting Co., USA), ce qui correspondait à des forces de 2,041 et 11,749 g (18,5 et 106,7 mN), respectivement. Les forces de flexion de ces filaments se sont révélées insuffisantes pour entraîner des réflexes de retrait nociceptifs chez les animaux normaux. Le nombre de retraits de patte provoqué par 10 essais a été établi pour chaque rat sur chaque patte arrière avant (état basal) et après l'induction de la mononeuropathie. Chez les rats normaux, les filaments de calibre faible (VFF 4,31) et de calibre élevé (VFF 5,07) ont provoqué en moyenne 1,3 ± 0,2 et 2,7 ± 0,3 réponses / 10 essais respectivement.

Après induction de la neuropathie, les deux filaments ont provoqué plus de 5 réponses / 10 essais.

La méthode décrite par Choi *et al*. (**1994**, *Behavioral signs of ongoing pain and cold allodynia in a rat model of neuropathic pain,* Pain, **59**:369-376) a été utilisée pour l'évaluation de l'allodynie au froid. Elle consiste à appliquer quelques gouttes (environ 50 *µ*l) d'une solution d'acétone sur la patte et de mesurer la durée de la réaction de retrait. Une demi-seconde et 20 secondes sont les valeurs choisies arbitrairement pour servir de seuil minimal et maximal respectivement.

La durée (D) de retrait de la patte (RP) en réponse à un rayon nociceptif de chaleur radiante orienté vers la surface plantaire a été établie chez le rat normal. L'augmentation de la DRP après induction de la mononeuropathie a été considérée comme une indication d'hyperpathie. Chaque rat a été soumis à deux tests RP par session, toutes les cinq minutes minimum.

Les manifestations neuropathiques ont été maximales sept à dix jours après induction de la neuropathie. Les effets des injections de PAT ont été testés pendant cette période, qui correspond au pic de la neuropathie.

### b) Protocole II : irritation chimique des afférences nociceptives

Ce protocole était basé sur les propriétés établies de la capsaicine, qui irrite de manière sélective un groupe spécifique de fibres afférentes (appelées " capsaicin sensitive primary afferents " ou CSPA, fibres afférentes primaires sensibles à la capsaïcine) connues pour provoquer une inflammation neurogène et pour transmettre l'information nociceptive (pour une revue, voir Szolcsanyi J., 1996, *Neurogeni c inflammation: reevaluation of axon reflex theory,* In: Geppetti P. and Holzer P. (Eds.), Neurogenic Inflammation, pp. 33-42. CRC Press, Boca Raton).

Notre groupe a mis au point deux méthodes : injection intra-plantaire (i. pl.) et intrapéritonéale (i. p.) de petites quantités de capsaïcine afin de provoquer des douleurs réversibles somatiques et viscérales respectivement.

### Injection intra-plantaire de capsaïcine

L'injection de capsaïcine (10 *µ*g dans 50 *µ*l d'une solution à 10 % de tween 20 dans de l'huile d'olive) a entraîné une hyperalgésie dont le pic a eu lieu trois à six heures après injection et qui a disparu au bout de 24 heures. L'hyperalgésie mécanique a été évaluée à l'aide du test de pression sur la patte (PP) qui a consisté à appliquer une pression constante de 0,2 kg/cm sur la partie dorsale de la patte arrière. Le temps écoulé entre l'application de la pression et le réflexe nociceptif de retrait de la patte a été considéré comme le temps de latence (ou seuil) de nociception mécanique. L'hyperalgésie thermique a été évaluée à l'aide du test de la plaque chaude (HP) et du test de l'immersion de la patte (PI).

Les tests HP et PI ont été menés comme dans l'exemple 1, la plaque chauffante ayant une température de 52, 5 ± 0, 3°C et le récipient d'eau chauffée, de 48 ± 0,3°C.

Les tests PP et PI ont été pratiqués l'un après l'autre sur les deux pattes arrières en respectant un intervalle de cinq minutes entre deux tests consécutifs.

Les rats ont été amenés au laboratoire une semaine avant l'injection afin qu'ils se familiarisent avec l'environnement et les tests ont été pratiqués sur les rats deux ou trois jours afin d'établir la valeur de départ pour chaque test avant tout traitement (description détaillée dans Kanaan S.A. et *al.,* **1996,** *Endotoxin-induced local inflammation and hyperalgesia in rats and mice: a new model for inflammatory pain,* Pain, **66**:373-379).

### Injection intrapéritonéale de capsaïcine

Ce test consistait à injecter (i. p.) 20 *µ*g de capsaïcine dans 100 *µ*l d'une solution de 10 % de tween 20 dans de l'huile d'olive, à des rats et à observer le comportement provoqué par ces injections. Une échelle comportementale de 4 niveaux a été conçue selon la méthode décrite par Giesler GJ *et al.,* (1976, *Inhibition of visceral pain by electrical stimulation of the periaqueductal gray matter, Pain* **2**:43-48).

Les niveaux ont été définis comme suit : 0 = comportement normal ; 1 = légère contraction des muscles abdominaux ; 2=contraction d'un seul côté et luxation de la hanche par enfoncement ; 3 = contraction importante des muscles abdominaux et extension des deux pattes arrière.

Pour l'évaluation du comportement des animaux, chaque rat a été placé dans une cage transparente placée elle-même au-dessus d'un miroir incliné à 45° pour une observation optimale.

Les comportements normaux et nociceptifs ont été enregistrés par un observateur à l'aide d'un polygraphe et le temps correspondant à chaque niveau sur une période de 30 minutes a été compté sur les enregistrements du polygraphe par un autre observateur. Aucun des deux observateurs ne connaissait l'injection pratiquée ni les effets attendus.

### Médicaments injectés

Le peptide PAT utilisé est le même que dans l'exemple 1. Le PAT a été dissous dans de l'eau distillée et administré en injection i. p. à une concentration et selon un planning appropriés selon le type d'expérience.

La capsaïcine (8-méthyl-N, Vanillyl-Nonanamide, Sigma #M1022) a été dissoute dans une solution à 10 % de tween 20 dans de l'huile d'olive et administrée en i.p. ou en i.pl. à la concentration appropriée.

### Analyse des données

Au cours des expériences portant sur des rats souffrant de neuropathie, l'allodynie et l'hyperpathie ont été évaluées dans chaque groupe d'animaux en référence à l'état basal établi avant induction de la mononeuropathie. Par exemple, la moyenne du nombre de retraits de la patte provoqués par chaque VFF a été calculée pour tous les rats de chaque groupe et la variation de cette moyenne a été déterminée après traitement par le PAT à des intervalles de temps différents après l'injection. La même méthode a été suivie pour l'allodynie au froid et l'hyperpathie à la chaleur.

Au cours des expériences impliquant l'injection i. pl. de capsaïcine, la moyenne des mesures effectuées à l'aide de chaque test de la douleur (PP, HP ou PI) a été calculée pour chaque groupe de rats avant l'injection de la capsaïcine (état basal) et à des intervalles de temps différents après l'injection (3, 6, 9 et 24 heures). Le PAT a été injecté (en i.p.) trente minutes avant la capsaïcine et les temps de latence des différents tests de la douleur ont été mesurés aux mêmes intervalles de temps après injection de la capsaïcine.

Pour l'injection i. p. de capsaïcine, le temps total correspondant à chaque niveau comportemental a été mesuré pour les animaux ayant reçu de la capsaïcine uniquement ou de la capsaïcine après le PAT. La variation des manifestations neuropathiques ou des tests de la douleur après les traitements ont été évalués par ANOVA puis par tests post hoc de Bonferroni. Les variations des scores de douleur avec ou sans traitement ont été évaluées par le test t de Student bilatéral en prenant comme limite de significativité 5 % (P<0,05).

### 2. Résultats

### Effets de l'injection de PAT sur les manifestations neuropathiques

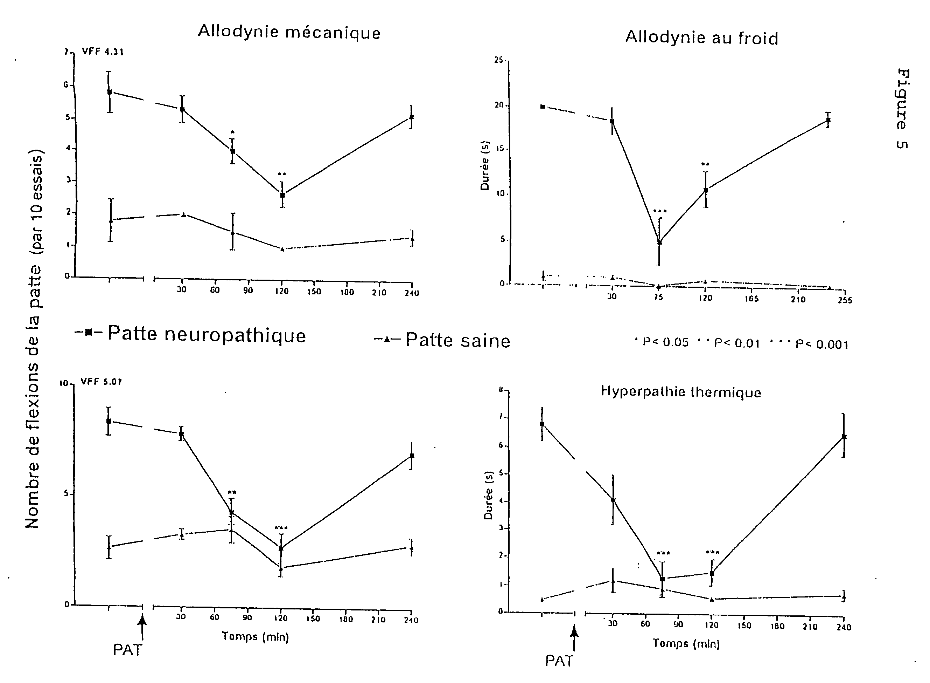

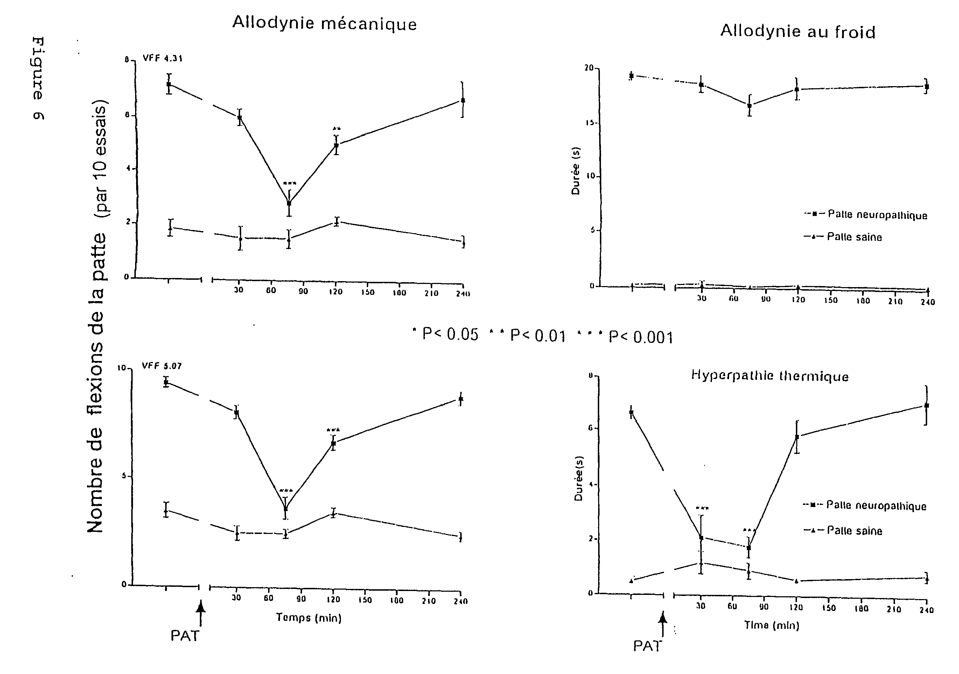

L'injection de PAT (5 *µ*g dans 100 *µ*l, i. p.) aux rats de deux groupes (n = 6 rats par groupe) exposés à une neuropathie provoquée par CCI ou SNI a entraîné une atténuation significative de toutes les manifestations neuropathiques (fig. 5 et 6). Cet effet a été maximal deux heures après injection pour l'allodynie mécanique. Il a été maximal 75 minutes après injection pour l'allodynie au froid et l'hyperpathie dans le modèle CCI et pour toutes les manifestations neuropathiques dans le modèle SNI. L'allodynie au froid n'a cependant été que modérément diminuée par le PAT dans le modèle SNI. La réversibilité des effets du PAT a été observée trois à quatre heures après injection.

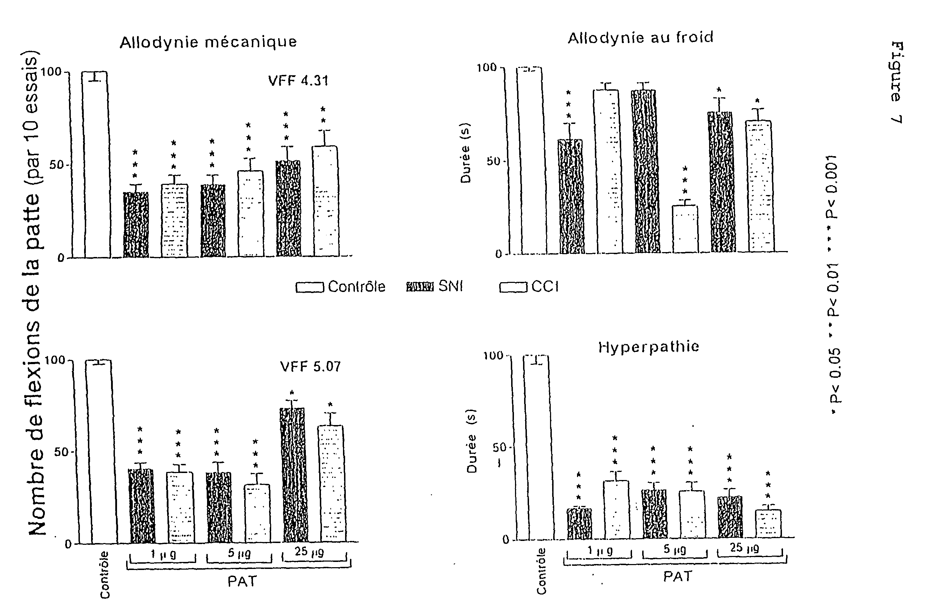

Les effets de doses de PAT de 1 et 25 *µ*g ont été évalués dans d'autres groupes de rats en suivant les deux modèles. La figure 7 montre que l'atténuation des manifestations neuropathiques a été maximale avec une dose de 5 *µ*g/rat.

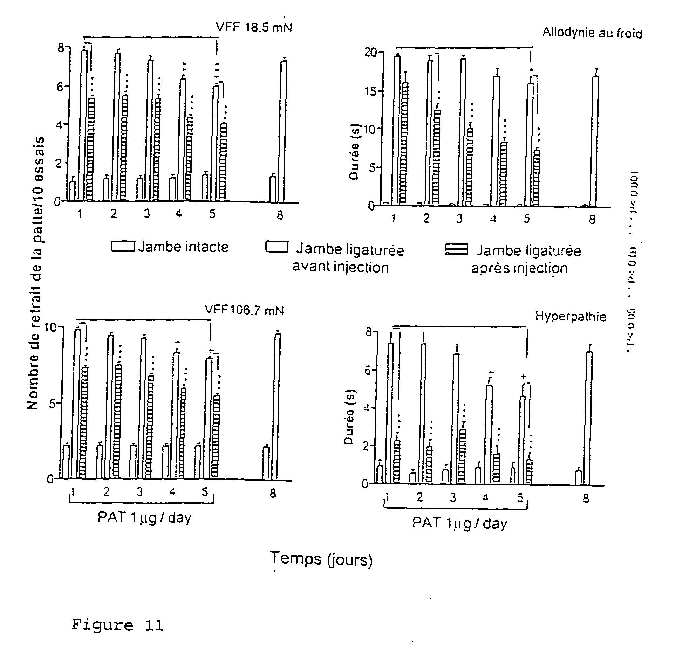

Un traitement quotidien par PAT (1*µ*g dans 100 µL) pendant 5 jours consécutifs, produit une diminution progressive des manifestations de l'allodynie ainsi qu'une potentialisation des effets de chaque injection. La preuve la plus évidente de cette potentialisation est l'inhibition accrue de l'allodynie au froid qui était peu modifiée par une seule injection de PAT (cf. Figure 11).

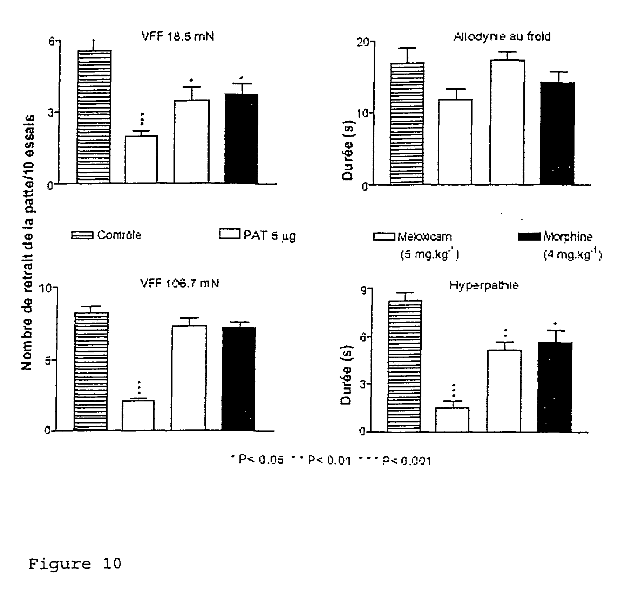

Les effets du traitement par PAT sur les manifestations de la neuropathie ont été comparés à ceux observés avec les injections, soit de meloxican (5mg/Kg, i.p.) soit de morphine(4mg/Kg, i.p.) à deux autres groupes de rats (n=6). Le traitement par PAT induit une réduction plus importante de l'allodynie tactile et de l'hyperalgésie thermique que celle observée avec les deux autres drogues, et ce à une dose beaucoup plus faible. La figure 10 illustre cette comparaison. Chaque drogue est injectée à un groupe différent (n=6) de rats exposés à une neuropathie provoquée par SNI. Toutes les mesures ont été réalisées au pic d'activité de chaque drogue (45-60 min après l'injection). Les contrôles correspondent aux mesures faites sur les rats exposés à une neuropathie avant tout traitement.

### Effets du traitement par le PAT sur l'hyperalgésie provoquée par l'injection i. pl. de capsaïcine

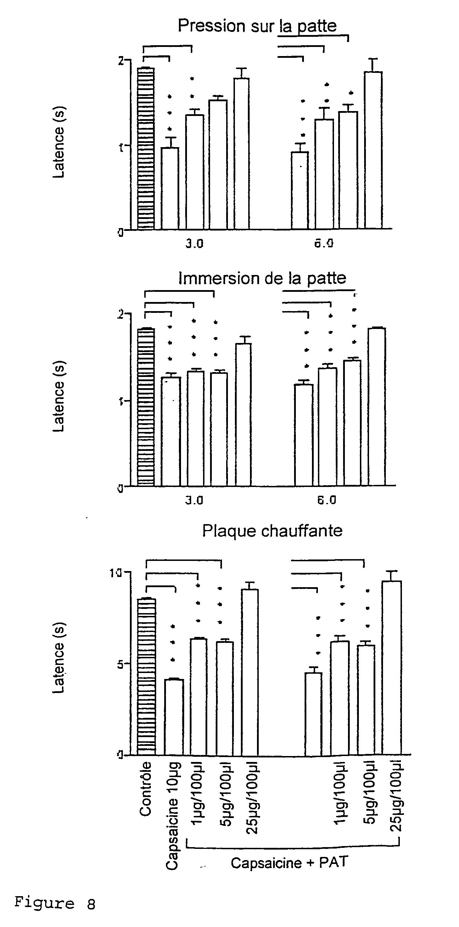

L'injection intra-plantaire de capsaïcine (10 *µ*g dans 50 *µ*l) a entraîné une diminution significative des temps de latence (hyperalgésie) observés lors des différents tests de la douleur. Cette diminution a été maximale au bout de trois à six heures et a disparu 24 heures après injection (Saadé N.E., Massaad C.A., Ochoa-Chaar C.I., Atweh S.F., Safieh-Garabedian B., Jabbur S.J. **2000**. *Possible contribution of neuropeptides and histamine to the hyperalgesia induced by intraplantar injection of capsaicin.* Eur. J. Neurosci. Abst. (suppl 11, Vol. 12 : 123). On a injecté à différents groupes de rats (n=5 rats dans chaque groupe) soit de la capsaïcine soit du PAT (i. p.) puis de la capsaïcine au bout de trente minutes, à des doses de 1,5 ou 25 *µ*g/rat. Le traitement préalable par le PAT a entraîné une atténuation dose dépendante de l'hyperalgésie provoquée par la capsaïcine (fig. 8). A la dose la plus élevée, l'injection de PAT a entraîné une prévention complète de l'hyperalgésie provoquée par la capsaïcine. L'injection d'une dose de 25 *µ*g de PAT n'a pas entraîné de modification significative des temps de latence observés lors des différents tests de la douleur (fig. 8, contrôles).

### Effets de l'injection de PAT sur la douleur viscérale provoquée par la capsaïcine

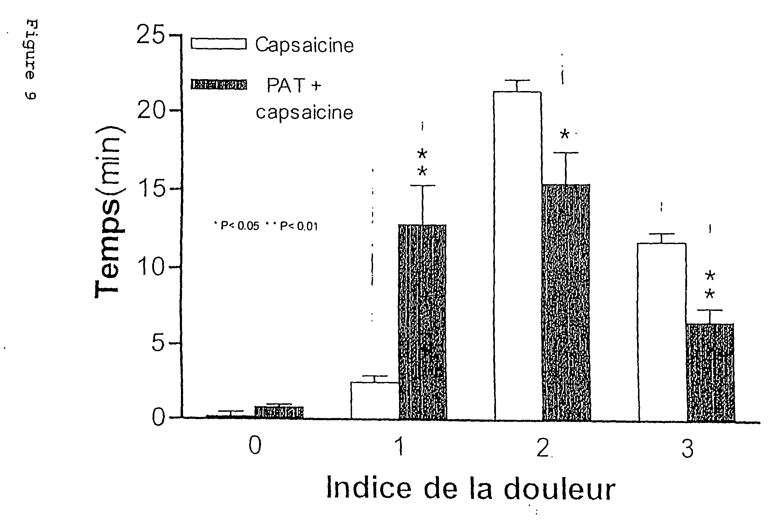

Des rats (n=6) ayant reçu une injection i. p. de capsaïcine (20 *µ*g dans 100 *µ*l) ont eu une réponse correspondant au niveau 0 pendant 0,2±0,3 minutes, au niveau 1 pendant 2,51 ± 0,45 minutes, au niveau 2 pendant 21,5 ± 0,8 minutes et au niveau 3 pendant 11,84 ± 0,43 minutes pendant une période d'observation totale de 36 minutes (fig. 9).

Dans un autre groupe de rats (n=6), l'injection de PAT (50*µ*g dans 200 *µ*l/rat) avant l'injection de capsaïcine (20 *µ*g, i.p.) a entraîné les scores de douleurs suivants : 0,82 ± 0,18 minutes correspondant au niveau 0, 12,89 ± 2,5 minutes correspondant au niveau 1, 15,62 ± 0,9 minutes correspondant au niveau 2 et 6,67 ± 0,09 minutes correspondant au niveau 3. Ainsi, le traitement préalable par le PAT a entraîné un déplacement significatif vers la gauche des scores de nociception provoquée par la capsaïcine (fig. 9).

### Exemple 3 : Utilisation de PAT pour fabriquer une solution injectable.

- PAT : 0,05 mg
- Eau distillée stérile exempte de pyrogènes : 1,0 ml.

Stérilisation par filtration, conditionnement en ampoule, flacons ou flacons à doses multiples.

Des voies d'administration possibles sont les injections intrapéritonéales, sous-cutanées, intracérébrales, intramusculaires et intradermiques.

## Revendications

1. Utilisation de peptides analogues de la thymuline sauf la thymuline, inactifs vis-à-vis du système immunitaire, ne comportant pas de zinc et présentant une activité anti-douleur, pour fabriquer un médicament destiné au traitement de la douleur.

2. Utilisation des peptides selon la revendication 1, **caractérisée en ce que** lesdits peptides présentent la séquence suivante :
X-Gln-Gly-Gly-Ser-Asn
dans laquelle X représente Ser, Lys-Ser, Ala-Lys-Ser, Glu-Ala-Lys-Ser, Gln-Ala-Lys-Ser, PyroGlu-Ala-Lys-Ser, ainsi que toute séquence dérivée comportant 1 ou 2 acides aminés modifiés, les modifications possibles étant de la nature suivante :
PyroGlu : D-PyroGlu, Glu, Gln
Gln : Z-Gln, D-Gln , Pro, Cys (S-CONH₂), Met(O), Glu, Glu (γ-cyano), Glu(γCS-NH₂), D-Glu, Asn, NorVal
Ala : D-Ala, Z-Ala, Ac-Ala
Lys : Arg, D-Lys, N-γ-Z-Lys, Lys(N⁶ acetyl), Orn, Har, 2-amino-hexanoyl, 2,6-diamino-hexynoyl, 2,6-diamino-hexenoyl, Hep, D-Lys(N⁶-acétyl)
Ser : Ala, (N-méthyl)Ser, D-Ser, Thr
Gly : Ala, Ser, D-Ala, D-Leu
Asn : CyanoAla, Thio-Asn, Asp, Gln, Glu, β-Ala-NH₂, D-Asn, Asn-NH₂
à l'exclusion de la séquence PyroGlu-Ala-Lys-Ser-Gln-Gly-Gly-Ser-Asn.

3. Utilisation des peptides selon les revendications 1 et 2, **caractérisée en ce que** les peptides sont les suivants :
(1) PyroGlu-Ala-Lys-Ser-Gln-Gly-Gly-Ser-Asp
(2) PyroGlu-Ala-Lys-Ala-Gln-Gly-Gly-Ser-Asn
(3) PyroGlu-Ala-Lys-Ser-Gln-Gly-Gly-Ser-Gln
(4) PyroGlu-Ala-Lys-Ser-Gln-Gly-Gly-Ser-β-Ala-NH₂
(5) PyroGlu-Ala-Lys-Ser-Gln-Gly-Gly-Ser-D-Asn
(6) PyroGlu-Ala-Lys-Ser-Gln-Gly-Gly-Ser-Asn-NH₂
(7) PyroGlu-Ala-Lys-Ser-Asn-Gly-Gly-Ser-Asn
(8) PyroGlu-Ala-Lys-Ser-Nva-Gly-Gly-Ser-Asn
(9) PyroGlu-Ala-Lys-Ser-Gln-Gly-Gly-Ala-Asp
(10) Gln-Ala-Lys-Ser-Gln-Gly-Gly-Ser-Asp
(11) Gln-Ala-Lys-Ala-Gln-Gly-Gly-Ser-Asn
(12) Gln-Ala-Lys-Ser-Gln-Gly-Gly-Ser-Gln
(13) Gln-Ala-Lys-Ser-Gln-Gly-Gly-Ser-β-Ala-NH₂
(14) Gln-Ala-Lys-Ser-Gln-Gly-Gly-Ser-D-Asn
(15) Gln-Ala-Lys-Ser-Gln-Gly-Gly-Ser-Asn-NH₂
(16) Gln-Ala-Lys-Ser-Asn-Gly-Gly-Ser-Asn
(17) Gln-Ala-Lys-Ser-Nva-Gly-Gly-Ser-Asn
(18) Gln-Ala-Lys-Ser-Gln-Gly-Gly-Ala-Asp
(19) Glu-Ala-Lys-Ser-Gln-Gly-Gly-Ser-Asp
(20) Glu-Ala-Lys-Ala-Gln-Gly-Gly-Ser-Asn
(21) Glu-Ala-Lys-Ser-Gln-Gly-Gly-Ser-Gln
(22) Glu-Ala-Lys-Ser-Gln-Gly-Gly-Ser-β-Ala-NH₂
(23) Glu-Ala-Lys-Ser-Gln-Gly-Gly-Ser-D-Asn
(24) Glu-Ala-Lys-Ser-Gln-Gly-Gly-Ser-Asn-NH₂
(25) Glu-Ala-Lys-Ser-Asn-Gly-Gly-Ser-Asn
(26) Glu-Ala-Lys-Ser-Nva-Gly-Gly-Ser-Asn
(27) Glu-Ala-Lys-Ser-Gln-Gly-Gly-Ala-Asp
(28) Ala-Lys-Ser-Gln-Gly-Gly-Ser-Asp
(29) Ala-Lys-Ala-Gln-Gly-Gly-Ser-Asn
(30) Ala-Lys-Ser-Gln-Gly-Gly-Ser-Gln
(31) Ala-Lys-Ser-Gln-Gly-Gly-Ser-β-Ala-NH₂
(32) Ala-Lys-Ser-Gln-Gly-Gly-Ser-D-Asn
(33) Ala-Lys-Ser-Gln-Gly-Gly-Ser-Asn-NH₂
(34) Ala-Lys-Ser-Asn-Gly-Gly-Ser-Asn
(35) Ala-Lys-Ser-Nva-Gly-Gly-Ser-Asn
(36) Ala-Lys-Ser-Gln-Gly-Gly-Ala-Asp
(37) Lys-Ser-Gln-Gly-Gly-Ser-Asp
(38) Lys-Ala-Gln-Gly-Gly-Ser-Asn
(39) Lys-Ser-Gln-Gly-Gly-Ser-Gln
(40) Lys-Ser-Gln-Gly-Gly-Ser-β-Ala-NH₂
(41) Lys-Ser-Gln-Gly-Gly-Ser-D-Asn
(42) Lys-Ser-Gln-Gly-Gly-Ser-Asn-NH₂
(43) Lys-Ser-Asn-Gly-Gly-Ser-Asn
(44) Lys-Ser-Nva-Gly-Gly-Ser-Asn
(45) Lys-Ser-Gln-Gly-Gly-Ala-Asp

4. Utilisation des peptides selon les revendications 1 à 3, **caractérisée en ce que** les peptides sont les suivants :
(19) Glu-Ala-Lys-Ser-Gln-Gly-Gly-Ser-Asp
(1) PyroGlu-Ala-Lys-Ser-Gln-Gly-Gly-Ser-Asp
(10) Gln-Ala-Lys-Ser-Gln-Gly-Gly-Ser-Asp
(28) Ala-Lys-Ser-Gln-Gly-Gly-Ser-Asp
(37) Lys-Ser-Gln-Gly-Gly-Ser-Asp

5. Utilisation des peptides selon les revendications 1 à 4, **caractérisée en ce que** le médicament possède des propriétés analgésiques et/ou anti-inflammatoires.

6. Utilisation des peptides selon les revendications 1 à 5 pour le traitement de la douleur engendrée par la migraine, la sciatique, la neuropathie et/ou les douleurs inflammatoires aigües ou chroniques.

7. Utilisation des peptides selon les revendications 1 à 6, **caractérisée en ce que** les doses administrées sont comprises entre 1µ g et 10 mg/kg.

8. Utilisation des peptides selon les revendications 1 à 7, **caractérisée en ce que** le médicament est formulé de manière à permettre une administration par voie parentérale ou nasale.

## Claims

1. Use of peptide analogues of thymulin, except thymulin, that are inactive relative to the immune system, and having anti-pain activity, for manufacturing a medicine for the treatment of pain.

2. Use of the peptides according to Claim 1, wherein said peptides have the following sequence :
X-Gln-Gly-Gly-Ser-Asn
wherein X represents Ser, Lys-Ser, Ala-Lys-Ser, Glu-Ala-Lys-Ser, Gln-Ala-Lys-Ser, PyroGlu-Ala-Lys-Ser, as well as any derived sequence comprising 1 or 2 modified amino acids, said possible modifications being of the following type:
PyroGlu : D-PyroGlu, Glu, Gln
Gln : Z-Gln, D-Gln , Pro, Cys (S-CONH₂), Met(O), Glu, Glu(γ-cyano), Glu(γCS-NH₂), D-Glu, Asn, NorVal
Ala : D-Ala, Z-Ala, Ac-Ala
Lys : Arg, D-Lys, N-γ-Z-Lys, Lys (N⁶-acetyl), Orn, Har, 2-amino-hexanoyl, 2,6-diaminohexynoyl, 2,6-diamino-hexenoyl, Hep, D-Lys (N⁶-acetyl)
Ser : Ala, (N-methyl)Ser, D-Ser, Thr
Gly : Ala, Ser, D-Ala, D-Leu
Asn : CyanoAla, Thio-Asn, Asp, Gln, Glu, β-Ala-NH₂, D-Asn, Asn-NH₂
with the exclusion of the PyroGlu-Ala-Lys-Ser-Gln-Gly-Gly-Ser-Asn sequence.

3. Use of peptides according to Claims 1 and 2, wherein the peptides are the following:
(1) PyroGlu-Ala-Lys-Ser-Gln-Gly-Gly-Ser-Asp
(2) PyroGlu-Ala-Lys-Ala-Gln-Gly-Gly-Ser-Asn
(3) PyroGlu-Ala-Lys-Ser-Gln-Gly-Gly-Ser-Gln
(4) PyroGlu-Ala-Lys-Ser-Gln-Gly-Gly-Ser-β-Ala-NH₂
(5) PyroGlu-Ala-Lys-Ser-Gln-Gly-Gly-Ser-D-Asn
(6) PyroGlu-Ala-Lys-Ser-Gln-Gly-Gly-Ser-Asn-NH₂
(7) PyroGlu-Ala-Lys-Ser-Asn-Gly-Gly-Ser-Asn
(8) PyroGlu-Ala-Lys-Ser-Nva-Gly-Gly-Ser-Asn
(9) PyroGlu-Ala-Lys-Ser-Gln-Gly-Gly-Ala-Asp
(10) Gln-Ala-Lys-Ser-Gln-Gly-Gly-Ser-Asp
(11) Gln-Ala-Lys-Ala-Gln-Gly-Gly-Ser-Asn
(12) Gln-Ala-Lys-Ser-Gln-Gly-Gly-Ser-Gln
(13) Gln-Ala-Lys-Ser-Gln-Gly-Gly-Ser-β-Ala-NH₂
(14) Gln-Ala-Lys-Ser-Gln-Gly-Gly-Ser-D-Asn
(15) Gln-Ala-Lys-Ser-Gln-Gly-Gly-Ser-Asn-NH₂
(16) Gln-Ala-Lys-Ser-Asn-Gly-Gly-Ser-Asn
(17) Gln-Ala-Lys-Ser-Nva-Gly-Gly-Ser-Asn
(18) Gln-Ala-Lys-Ser-Gln-Gly-Gly-Ala-Asp
(19) Glu-Ala-Lys-Ser-Gln-Gly-Gly-Ser-Asp
(20) Glu-Ala-Lys-Ala-Gln-Gly-Gly-Ser-Asn
(21) Glu-Ala-Lys-Ser-Gln-Gly-Gly-Ser-Gln
(22) Glu-Ala-Lys-Ser-Gln-Gly-Gly-Ser-β-Ala-NH₂
(23) Glu-Ala-Lys-Ser-Gln-Gly-Gly-Ser-D-Asn
(24) Glu-Ala-Lys-Ser-Gln-Gly-Gly-Ser-Asn-NH₂
(25) Glu-Ala-Lys-Ser-Asn-Gly-Gly-Ser-Asn
(26) Glu-Ala-Lys-Ser-Nva-Gly-Gly-Ser-Asn
(27) Glu-Ala-Lys-Ser-Gln-Gly-Gly-Ala-Asp
(28) Ala-Lys-Ser-Gln-Gly-Gly-Ser-Asp
(29) Ala-Lys-Ala-Gln-Gly-Gly-Ser-Asn
(30) Ala-Lys-Ser-Gln-Gly-Gly-Ser-Gln
(31) Ala-Lys-Ser-Gln-Gly-Gly-Ser-a-Ala-NH₂
(32) Ala-Lys-Ser-Gln-Gly-Gly-Ser-D-Asn
(33) Ala-Lys-Ser-Gln-Gly-Gly-Ser-Asn-NH₂
(34) Ala-Lys-Ser-Asn-Gly-Gly-Ser-Asn
(35) Ala-Lys-Ser-Nva-Gly-Gly-Ser-Asn
(36) Ala-Lys-Ser-Gln-Gly-Gly-Ala-Asp
(37) Lys-Ser-Gln-Gly-Gly-Ser-Asp
(38) Lys-Ala-Gln-Gly-Gly-Ser-Asn
(39) Lys-Ser-Gln-Gly-Gly-Ser-Gln
(40) Lys-Ser-Gln-Gly-Gly-Ser-β-Ala-NH₂
(41) Lys-Ser-Gln-Gly-Gly-Ser-D-Asn
(42) Lys-Ser-Gln-Gly-Gly-Ser-Asn-NH₂
(43) Lys-Ser-Asn-Gly-Gly-Ser-Asn
(44) Lys-Ser-Nva-Gly-Gly-Ser-Asn
(45) Lys-Ser-Gln-Gly-Gly-Ala-Asp

4. Use of the peptides according to Claims 1 to 3, wherein the peptides are the following:
(19) Glu-Ala-Lys-Ser-Gln-Gly-Gly-Ser-Asp
(1) PyroGlu-Ala-Lys-Ser-Gln-Gly-Gly-Ser-Asp
(10) Gln-Ala-Lys-Ser-Gln-Gly-Gly-Ser-Asp
(28) Ala-Lys-Ser-Gln-Gly-Gly-Ser-Asp
(37) Lys-Ser-Gln-Gly-Gly-Ser-Asp

5. Use of the peptides according to Claims 1 to 4, wherein the medicine has analgesic and / or antiinflammatory properties.

6. Use of the peptides according to Claims 1 to 5, for the treatment of pain resulting from migraine, sciatica, neuropathy and / or acute or chronic inflammatory pain.

7. Use of the peptides according to Claims 1 to 6, wherein the doses administered are between 1 µg and 10 mg/kg.

8. Use of the peptides according to Claims 1 to 7, wherein the medicine is formulated so as to permit administration by parenteral or nasal route.

## Patentansprüche

1. Verwendung von Peptiden, die analog zu Thymulin sind, außer Thymulin, und die inaktiv gegenüber dem Immunsystem sind, kein Zink umfassen und eine Anti-Schmerz-Aktivität aufweisen, zur Herstellung eines Medikaments zur Behandlung von Schmerzen.

2. Verwendung der Peptide gemäß Anspruch 1, **dadurch gekennzeichnet, dass** die Peptide die folgende Sequenz aufweisen:
X-Gln-Gly-Gly-Ser-Asn,
wobei X für Ser, Lys-Ser, Ala-Lys-Ser, Glu-Ala-Lys-Ser, Gln-Ala-Lys-Ser, PyroGlu-Ala-Lys-Ser sowie jede davon abgeleitete Sequenz steht, die 1 oder 2 modifizierte Aminosäuren umfasst, wobei die möglichen Modifikationen von folgender Art sind:
PyroGlu: D-PyroGlu, Glu, Gln
Gln: Z-Gln, D-Gln, Pro, Cys (S-CONH₂), Met(O), Glu, Glu(γ-cyano), Glu(γCS-NH₂), D-Glu, Asn, NorVal
Ala : D-Ala, Z-Ala, Ac-Ala
Lys: Arg, D-Lys, N-γ-Z-Lys, Lys(N⁶-acetyl), Orn, Har, 2-Aminohexanoyl, 2,6-Diaminohexinoyl, 2,6-Diamino-hexenoyl, Hep, D-Lys(N⁶-acetyl)
Ser: Ala, (N-methyl)Ser, D-Ser, Thr
Gly: Ala, Ser, D-Ala, D-Leu
Asn: CyanoAla, Thio-Asn, Asp, Gln, Glu, β-Ala-NH₂, D-Asn, Asn-NH₂
mit Ausnahme der Sequenz PyroGlu-Ala-Lys-Ser-Gln-Gly-Gly-Ser-Asn.

3. Verwendung der Peptide gemäß Anspruch 1 und 2, **dadurch gekennzeichnet, dass** die Peptide die folgenden sind:
(1) PyroGlu-Ala-Lys-Ser-Gln-Gly-Gly-Ser-Asp
(2) PyroGlu-Ala-Lys-Ala-Gln-Gly-Gly-Ser-Asn
(3) PyroGlu-Ala-Lys-Ser-Gln-Gly-Gly-Ser-Gln
(4) PyroGlu-Ala-Lys-Ser-Gln-Gly-Gly-Ser-β-Ala-NH₂
(5) PyroGlu-Ala-Lys-Ser-Gln-Gly-Gly-Ser-D-Asn
(6) PyroGlu-Ala-Lys-Ser-Gln-Gly-Gly-Ser-Asn-NH₂
(7) PyroGlu-Ala-Lys-Ser-Asn-Gly-Gly-Ser-Asn
(8) PyroGlu-Ala-Lys-Ser-Nva-Gly-Gly-Ser-Asn
(9) PyroGlu-Ala-Lys-Ser-Gln-Gly-Gly-Ala-Asp
(10) Gln-Ala-Lys-Ser-Gln-Gly-Gly-Ser-Asp
(11) Gln-Ala-Lys-Ala-Gln-Gly-Gly-Ser-Asn
(12) Gln-Ala-Lys-Ser-Gln-Gly-Gly-Ser-Gln
(13) Gln-Ala-Lys-Ser-Gln-Gly-Gly-Ser-β-Ala-NH₂
(14) Gln-Ala-Lys-Ser-Gln-Gly-Gly-Ser-D-Asn
(15) Gln-Ala-Lys-Ser-Gln-Gly-Gly-Ser-Asn-NH₂
(16) Gln-Ala-Lys-Ser-Asn-Gly-Gly-Ser-Asn
(17) Gln-Ala-Lys-Ser-Nva-Gly-Gly-Ser-Asn
(18) Gln-Ala-Lys-Ser-Gln-Gly-Gly-Ala-Asp
(19) Glu-Ala-Lys-Ser-Gln-Gly-Gly-Ser-Asp
(20) Glu-Ala-Lys-Ala-Gln-Gly-Gly-Ser-Asn
(21) Glu-Ala-Lys-Ser-Gln-Gly-Gly-Ser-Gln
(22) Glu-Ala-Lys-Ser-Gln-Gly-Gly-Ser-β-Ala-NH₂
(23) Glu-Ala-Lys-Ser-Gln-Gly-Gly-Ser-D-Asn
(24) Glu-Ala-Lys-Ser-Gln-Gly-Gly-Ser-Asn-NH₂
(25) Glu-Ala-Lys-Ser-Asn-Gly-Gly-Ser-Asn
(26) Glu-Ala-Lys-Ser-Nva-Gly-Gly-Ser-Asn
(27) Glu-Ala-Lys-Ser-Gln-Gly-Gly-Ala-Asp
(28) Ala-Lys-Ser-Gln-Gly-Gly-Ser-Asp
(29) Ala-Lys-Ala-Gln-Gly-Gly-Ser-Asn
(30) Ala-Lys-Ser-Gln-Gly-Gly-Ser-Gln
(31) Ala-Lys-Ser-Gln-Gly-Gly-Ser-β-Ala-NH₂
(32) Ala-Lys-Ser-Gln-Gly-Gly-Ser-D-Asn
(33) Ala-Lys-Ser-Gln-Gly-Gly-Ser-Asn-NH₂
(34) Ala-Lys-Ser-Asn-Gly-Gly-Ser-Asn
(35) Ala-Lys-Ser-Nva-Gly-Gly-Ser-Asn
(36) Ala-Lys-Ser-Gln-Gly-Gly-Ala-Asp
(37) Lys-Ser-Gln-Gly-Gly-Ser-Asp
(38) Lys-Ala-Gln-Gly-Gly-Ser-Asn
(39) Lys-Ser-Gln-Gly-Gly-Ser-Gln
(40) Lys-Ser-Gln-Gly-Gly-Ser-β-Ala-NH₂
(41) Lys-Ser-Gln-Gly-Gly-Ser-D-Asn
(42) Lys-Ser-Gln-Gly-Gly-Ser-Asn-NH₂
(43) Lys-Ser-Asn-Gly-Gly-Ser-Asn
(44) Lys-Ser-Nva-Gly-Gly-Ser-Asn
(45) Lys-Ser-Gln-Gly-Gly-Ala-Asp.

4. Verwendung der Peptide gemäß den Ansprüchen 1 bis 3, **dadurch gekennzeichnet, dass** die Peptide die folgenden sind:
(19) Giu-Ala-Lys-Ser-Gin-Gly-Gly-Ser-Asp
(1) PyroGlu-Ala-Lys-Ser-Gln-Gly-Gly-Ser-Asp
(10) Gln-Ala-Lys-Ser-Gln-Gly-Gly-Ser-Asp
(28) Ala-Lys-Ser-Gln-Gly-Gly-Ser-Asp
(37) Lys-Ser-Gln-Gly-Gly-Ser-Asp.

5. Verwendung der Peptide gemäß den Ansprüchen 1 bis 4, **dadurch gekennzeichnet, dass** das Medikament analgetische und/oder entzündungshemmende Eigenschaften hat.

6. Verwendung der Peptide gemäß den Ansprüchen 1 bis 5 zur Behandlung von Schmerzen, die durch Migräne, Ischias, Neuropathie und/oder akute oder chronische Entzündungsschmerzen hervorgerufen werden.

7. Verwendung der Peptide gemäß den Ansprüchen 1 bis 6, **dadurch gekennzeichnet, dass** die verabreichten Dosen 1 µg bis 10 mg/kg betragen.

8. Verwendung der Peptide gemäß den Ansprüchen 1 bis 7, **dadurch gekennzeichnet, dass** das Medikament für die parenterale oder nasale Verabreichung zubereitet wird.
